(19) Europäisches Patentamt

European Patent Office

Office européen des brevets

(11) **EP 1 615 030 A1**

(12) **EUROPEAN PATENT APPLICATION**

(43) Date of publication:
**11.01.2006 Bulletin 2006/02**

(51) Int Cl.:
*G01N 33/24* *(2006.01)*    *G01N 15/08* *(2006.01)*

(21) Application number: **04076931.7**

(22) Date of filing: **05.07.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **NEDERLANDSE ORGANISATIE VOOR TOEGEPAST-NATUURWETENSCHAPPELIJK ONDERZOEK TNO
2628 VK Delft (NL)**

(72) Inventors:
• **Hofstee, Cornelis
3985 PD Werkhoven (NL)**
• **Swarbrick, Gareth
NSW 2052 Sydney (AU)**

(74) Representative: **Winckels, Johannes Hubertus F. et al
Vereenigde
Johan de Wittlaan 7
2517 JR Den Haag (NL)**

(54) **Method for predicting final saturation of an organic liquid phase in a porous medium**

(57)    The invention provides a method for predicting final organic saturation in a porous medium comprising an aqueous phase, a gaseous phase and an organic liquid phase. The invention further provides a process for recovering an organic liquid phase from a subsurface formation or prediction of the amount of organic contaminants in the vadose zone, in which process use is made of a method according to the present invention for predicting the quantity of final organic liquid phase present in the subsurface formation.

**Description**

The present invention relates to a method for predicting final organic saturation in a porous medium.

In the field of oil recovery there is nowadays an increasing need to determine the maximum amount of oil that can be recovered from a particular oil reservoir. Additionally, there is a clear need to determine the amount of organic contaminants present in contaminated subsurfaces.

The various models that are currently applied for these two purposes are based on multi-fluid simulators for predicting final saturation in a porous medium. As the skilled person will appreciate, final saturation in a three-fluid porous medium system consists of the summation of the saturations of water-entrapped globules and films or microlenses (detached saturation) on top of the water-gas interface. A particular limitation of current continuum-based numerical simulators in the area of multi-fluid flow and transport is, however, their inability to account for non-spreading behaviour of the organic phase in porous media that contain three fluids, viz. an aqueous phase, an organic phase and a gaseous phase. It is believed that this limitation is due to the use of the Leverett and Lewis (1941) concept to estimate three-fluid aqueous-organic and gaseous phase retention curves from two-fluid retention measurements. The basic assumption underlying the Leverett concept is that the intermediate wetting fluid forms a continuous film on water-air interfaces. It has however be found by several researchers that this assumption is only valid until the saturation of the organic phase drops to a certain level at which the flow of non-spreading oils ceases, while spreading oils continue to flow, as predicted by the numerical simulators. The effect is that the numerical simulators may underestimate the amount of organic contaminants, which are retained in the unsaturated zone and overestimate the amount of oil, which can be produced in porous media under three-fluids conditions. In other words, the current methods for predicting final organic saturation in a porous medium may not always enable one to determine accurately the maximum amount of oil that can be recovered from an oil reservoir or to determine adequately the amount of organic contaminants present in a contaminated subsurface.

Object of the present invention is to provide an improved method for predicting final organic saturation in a porous medium, enabling a more accurate determination of the amount of oil in an oil reservoir or the amount of organic contaminants present in a contaminated subsurface.

Surprisingly, it has now been found that this object can be established when use is made of a particular sequence of process steps.

Accordingly, the present invention relates to a method for predicting final organic saturation in a porous medium comprising an aqueous phase, a gaseous phase and an organic liquid phase, which method comprises the steps of:

a) providing a sample of the porous medium;

b) determining the relationship between the permeability, fluid saturation and capillary pressure of the sample;

c) determining the critical detached organic saturation (CDOS) of the sample at a particular aqueous or gaseous saturation, whereby the CDOS is defined as being the critical continuous organic saturation at the continuous aqueous gas interface at which the relative permeability of the gaseous phase of the sample falls abruptly to zero;

d) estimating the interfacial area between the continuous aqueous and gaseous phases as a function of the aqueous or gaseous saturation;

e) determining the ratio of the CDOS over the area of the interface between the aqueous and gaseous phases;

f) performing a simulation of the distribution of the aqueous and gaseous phases using a continuum based multi-fluid flow model;

g) determining for each time step and note of the simulation whether the organic liquid phase saturation exceeds the CDOS at the particular aqueous or gaseous saturation;

h) set the relative permeability of the organic liquid phase to zero, when the CDOS has been reached; and

i) continue the simulation of the distribution of the aqueous and gaseous phases and maintain the relative permeability of the organic liquid phase at zero as long as the CDOS is not exceeded.

In step (a) of the method according to the present invention a sample of the porous medium is provided. Such a sample may comprise an undisturbed soil sample, a rock core or an artificially packed column or flow cell.

A preferred method comprises obtaining representative samples of the porous media in oil reservoirs or in the vadose zone, which is under consideration and to recreate the porous media in a 1-D column or flow cell. Alternatively, it may be required to extract complete porous medium samples from the subsurface, for example, in the case of consolidated geological formations.

In step (b) of the method according to the invention the relationship between the permeability, fluid saturation and capillary pressure of the sample is determined. Reliable determination of these properties is essential for input in continuum-based numerical simulators to obtain meaningful predictions.

A suitable way to estimate $k_{nw}(S)$ and $k_w(S)$ relationships (see ref. 2) is to substitute fitted $P_c(S)$ relationships on the basis of measured data and the theory of Brooks and Corey (see ref. 1), into the pore-size model of Purcell (see ref.

10). Also, a combination of the Van Genuchten (see ref. 11) retention function and the Mualem (see ref. 7) pore-size distribution model can suitably be used.

Application of the method presented by Kaluarachi and Parker (see ref. 5) and substitution of the Brooks and Corey (see ref. 1) (BC) relationships give the relative aqueous permeability, $k_{rl}$, and the relative NAPL permeability, $k_{rn}$ as:

$$k_{rl} = \overline{S}_l^{\left(\frac{2+3\lambda}{\lambda}\right)} \tag{1}$$

$$k_{rn} = \overline{S}_{nf}^{\,2}\left[\overline{\overline{S}}_l^{\left(\frac{2+\lambda}{\lambda}\right)} - \overline{\overline{S}}_l^{\left(\frac{2+\lambda}{\lambda}\right)}\right]$$

A variety of methods have been developed to determine the aforementioned constitutive relationships. However, there is no single method, which is best suited for all circumstances. A preferred method in accordance with the present invention comprises the use of a single 1-D column, packed as uniformly as possible with a porous medium. After establishing fully water-saturated conditions, preferably a constant head method is used to measure the saturated hydraulic conductivity. Subsequently, the columns can be drained. After the system has been allowed to come to hydrostatic equilibrium (no flow conditions), the columns are scanned to determine the water saturation as a function of elevation. Since the condition of hydrostatic equilibrium is fulfilled, the capillary pressure at each elevation can be calculated. The constitutive relationships for water-air systems can now be determined, preferably by using the BC relationships. These relationships can be adjusted for other combinations of pairs of fluids (for example water-oil) by using preferably measured values of the viscosity, density and interfacial tension. Extensions to 3 fluids combinations can be reached by applying the concept put forward by Leverett and Lewis (see ref. 6). Using the same scaling principles, it is possible to correct for, for example, temperature effects.

Techniques suitable for obtaining capillary pressure-saturation curves in the absence of a scanning technique such as a gamma radiation or x-ray system include the use of suction apparatuses and pressure cells (vadose zone hydrology, environmental engineering) and methods involving mercury intrusion and centrifuges (petroleum industry).

In step (c) of the method according to the present invention, the critical detached organic saturation (CDOS) of the samples determined at a particular aqueous or gaseous saturation, whereby the CDOS is defined as being the critical continuous organic saturation at the continuous aqueous gas interface at which 5the relative permeability of the gaseous phase of the sample falls abruptly to zero.

The CDOS has to be determined in a porous medium containing a continuous aqueous and gaseous phase. A continuous organic phase is introduced in this flow domain and allowed to drain until it stops flowing. At that time the saturation of the water and the organic phase has to be determined. This can be done by detector equipment such as, for example, dual-energy gamma radiation systems or indirectly using, for example, extraction methods. Examples of methods to find the CDOS at specific water saturations can be found in Hofstee et al. (see ref. 3); Hofstee et al. (see ref. 4); and Oostrom et al. (see ref. 8).

For the aforementioned determination of the CDOS, it is paramount to make a distinction between the saturation of water-entrapped organic phase and that of the organic present, which is present in micro-lenses on top of the water-air interface. In situations where entrapment has occurred during the experiment (due to imbibition of water) the amount of entrapped phase should be subtracted from the total retained organic saturation. A procedure to apply this correction is described in Hofstee et al. (see ref. 3). Preferably, a 1-D column is used for this purpose, which is packed with one or several layers, containing representative samples of the porous media in oil reservoirs or in the vadose zone, which is under consideration. Extraction methods can, of course, be considered when the aforementioned specialized detection is not available.

In step (d) of the method according to the present invention the interfacial area between the continuous aqueous and gaseous phases is estimated as a function of the aqueous saturation. In accordance with the present invention, it has now been found that detached NAPL saturation is a direct function of the gas-aqueous interfacial area within the porous media. This interfacial area increases with a decreasing aqueous saturation. Since the interfacial area is not a readily available quantity for a real porous media such as soil, an approximate method is required to estimate this area based upon the aqueous saturation. One such estimation method has been developed by Oostrom et al. (see ref. 9), whereby the NAPL- aqueous interfacial area, $A_{nl}^f$, is estimated as:

$$A_{nl}^f = \frac{(1-S_m)n_D}{\sigma_{nl}} \int_{\bar{S}_l}^{1} P_{c\,nl}\, d\bar{\bar{S}}_l \qquad\qquad (2$$

where $n_D$ is the porosity, $g$ is the gravitational constant, $\rho_l$ is the liquid density, and $P_{cnl}$ is the aqueous-NAPL capillary pressure.

To solve for $A_{nl}^f$ requires the use of a $P_c$-$S$ relationship. Using the relationship proposed by Brooks and Corey (see ref. 1) the integrated form of equation (2) is:

$$A_{nl}^f = \frac{(1-S_m)n_D P_d}{\sigma_{nl}\left(1-\frac{1}{\lambda}\right)} \left(1 - \bar{\bar{S}}_l^{\left(1-\frac{1}{\lambda}\right)}\right) \qquad\qquad (3$$

where $P_d$ is the air entry pressure, $\lambda$ the pore-size distribution parameters, $n_d$ the diffusive porosity, $S_m$ the irreducible water content respectively, and $\sigma_{nl}$ is the nonwetting-wetting fluid interfacial tension.

Other $P_c$-$s$ relationships can also suitably be used. However, such functions may need to be integrated numerically, which complicates the procedure. For this reason, and the fact that the Brooks and Corey relationships describe a distinct entry pressure, the BC relationships are preferably used.

In step (e) the ratio of the CDOS over the area of the interface between the aqueous and gaseous phases is determined.

It has now been found that the $\bar{S}_{nd}^c$ (CDOS) is a direct function of the $A_{nl}^f$ which results in:

$$\frac{\bar{S}_{nd}^c}{A_{nl}^f} = B \qquad\qquad (4)$$

where B is a constant, which represents the NAPL saturation, which can be stored as detached NAPL saturation on a unit area of water-air interface ($1/m^2$).

It should be noted that the actual detached NAPL saturation depends on the available NAPL and may be equal to or les than $\bar{S}_{nd}^c$.

The definition of B indicates that B may be more or less a constant for various porous media. As the $A_{nl}^f$ is a function of a number of constants and the capillary pressure -saturation curve, it is now possible to make an estimate of the CDOS using $P_c$-S curves.

The preferred approach is currently a dedicated measurement of CDOS for each porous medium under consideration.

In step (f) a simulation of the distribution of the phases is performed using a continuum based multi-fluid flow model.

A convenient way to implement the concept of CDOS in a numerical simulator is to rewrite equation (4) as

$$\bar{S}_{nd}^c = \bar{S}_{nd}^{max}\left(1 - \bar{\bar{S}}_l^{\left(1-\frac{1}{\lambda}\right)}\right) \qquad\qquad (5)$$

where $\bar{\bar{S}}$ is the apparent aqueous saturation.

In step (g) it is determined for each time step and note of the simulation whether the organic phase saturation exceeds the CDOS at the particular aqueous or

gaseous saturation. This can suitably be established by comparing the simulated free organic saturations with the CDOS, which is calculated for each node and time step using the simulated aqueous saturations.

In step (h) of the method according to the present invention the relative permeability of the organic phase is set to zero, when the CDOS has been reached.

It will be understood that NAPL at free saturations, which are at or below critical detached saturation, is discontinuous and therefore cannot flow. Effectively, the relatively permeability will under such circumstances be zero. To accommodate this effect changes must be made to the estimation of relative permeability. It will further be understood that at saturations above critical detached saturation, all free NAPL participates in flow and that therefore equation (1) applies. $\overline{S}_i$ in equation (1) is defined by:

$$\overline{S}_{nf} = \overline{S}_n - \overline{S}_{nt} \qquad (6)$$

where the subscripts nf, n and nt stand for free-NAPL, NAPL and water-entrapped NAPL, respectively.

From this it follows that once the NAPL film has become discontinuous, the flow will cease. In accordance with the present invention it has therefore now been found that there is a discrete change from free flowing NAPL to no flow once residual NAPL saturation is reached. This surprising finding can is mathematically be represented by:

$$k_{rn} = \overline{S}_{nf}^{\ 2} \left[ \overline{\overline{S}}_t^{\left(\frac{2+\lambda}{\lambda}\right)} - \overline{\overline{S}}_l^{\left(\frac{2+\lambda}{\lambda}\right)} \right] \quad \overline{S}_{nf} > \overline{S}_{nd}^c \text{ and}$$
$$k_{rn} = 0 \qquad\qquad \overline{S}_{nf} \le \overline{S}_{nd}^c \qquad\qquad\qquad (7)$$

Direct implementation of equation (7) may, however, cause the simulator's iterative solver to be unable to converge, which could be due to the step function nature of the equation. Preferably, therefore, a smooth function is used. In accordance with the present invention it has been found that the following smooth function is most preferably used:

$$k_{rn}' = \left(1 - \left[\frac{S_{nd}}{S_n}\right]^4\right) k_{rn} \qquad\qquad (8)$$

When use is made of this particular function, the smoothed value of the relative NAPL permeability, $k_{rn}'$ , is within 90 % of $k_{rn}$ when $S_n$ is greater than 1.7 * $S_{nd}$. In addition, $k_{rn}'$ drops to zero when $S_n$ equals $S_{nd}$ resulting in a smooth transition between the point of detachment.

In view of this finding, in step (i), the simulation of the simulation of the distribution of the aqueous and gaseous phases is continued and the relative permeability of the organic phase is maintained at zero as long as the CDOS is not exceeded.

Examples

Example 1

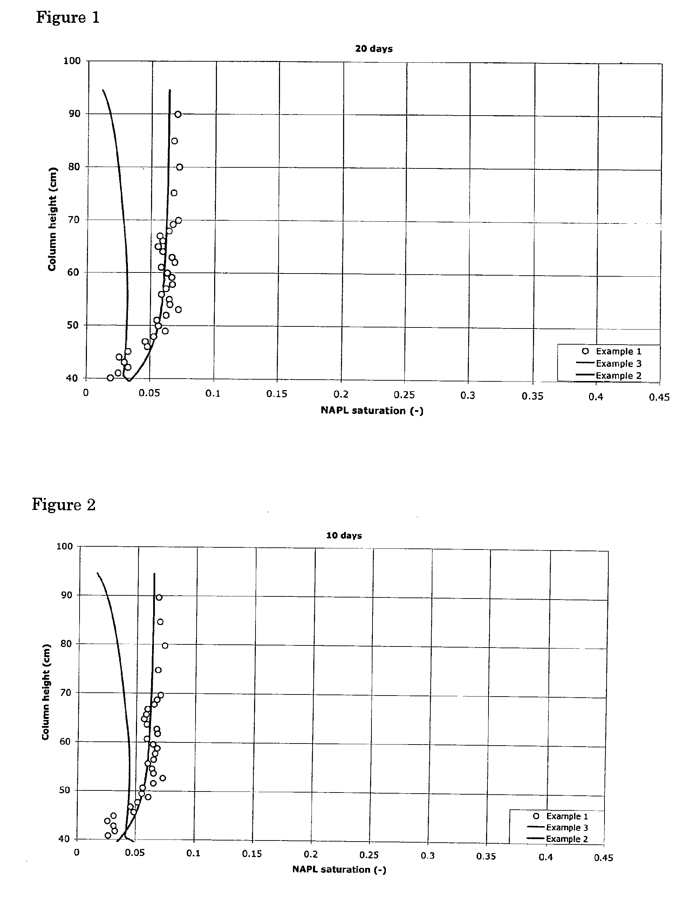

Experiments which are very relevant to the validation of the current invention, were published in Oostrom et al. (see ref. 8) These experiments were conducted in a 95-cm-long glass column with a 7.5-m diameter. The main objectives for these experiments were to determine the hydraulic properties and the carbon tetrachloride retention characteristics. The porous media used in this study was a fine-grained (70 mesh) sand (Unimin Corporation, Le Sueur, MN). The column was homogeneously packed with the sand under water-saturated conditions. After completion of the packing, a constant-head method was used to determine the hydraulic conductivity of the sands. Average porosity values and associated standard deviations were obtained using a dual-energy gamma radiation system. Denoting the bottom of the column at $z = 0$ cm, the column was calibrated for gamma measurements at 88 locations with a 1-cm spacing between $z = 5$ and $z = 92$ cm. The column was subsequently drained at a rate of 10 cm/hours to a final water table location at $z = 5$ cm. After allowing drainage for 2 days, the column was scanned to determine the water saturation as a function of elevation. After the scans, 0.5 ml of carbon tetrachloride was injected at $z = 80$ cm with a rate of 0.05 ml/minute. The column was capped with a glass cover to reduce the escape of vapors. The covers have a 0.1-cm pinhole to maintain atmospheric pressure inside the system. A small container with water was placed on top of the sand to maximize the water vapor concentration at the boundary, thus reducing water evaporation from the porous medium. After injection of the carbon tetrachloride, water drainage from the column was recorded. Another gamma scan to determine water saturations was obtained 2 days after the DNAPL was injected. The counting time per location was 90 seconds. Next, $CCl_4$ was added to the column setup, at a rate of 1.0 ml/min at the top of the sand ($z$ = 95 cm). The injected amount was 120.0 ml. The DNAPL was dyed with 0.1 g/L Sudan IV dye. Gamma scans to determine $CCl_4$ saturations were obtained after 12 hours, 10 and 20 days since initiating of the injection. During the more dynamic initial stages, gamma readings were obtained at 5-cm intervals only. The measured profiles of the measured NAPL saturations against elevation are shown in Figures 1-3.

Example 2

A modified STOMP method in accordance with the present invention was carried out as follows. The column experiment of Example 1 was simulated with a modified version of the oil mode of the STOMP (Subsurface Transport Over Multiple Phases) code (see ref. 12 and ref. 13). The original STOMP was developed by the Pacific Northwest National Laboratories in Richland, WA. This existing STOMP code was modified to include:

   1) The prediction of the detached NAPL saturation - equation 5.

   2) Changes to the calculation of the relative NAPL permeability according to equations 7 and 8.

   3) Release of the existing constraints with respect to interfacial tensions.

The permeability and the relationship between the capillary pressure and the aqueous saturation were taken from the measurement in Example 1. These relationships, which pertain to water-gaseous systems, were made applicable to three-fluid systems using measured values of the interfacial tensions, the densities and viscosities of the various fluids. All measurements were conducted under the condition of chemical equilibrium between the various fluids. The maximum entrapped and detached NAPL saturations were also based upon the experimental observation, which was described in Example 1.The 1-D computation domain consisted of 190 0.5 cm cells. At the bottom, a constant aqueous pressure was prescribed, consistent with a water table position at z=5 cm. Zero aqueous flux boundary conditions were specified at the top and at the perimeter of the column. Zero flux boundaries were specified for $CCl_4$ on all boundaries except the 2 hours of infiltration, when the NAPL was allowed to infiltrate from the top into the domain using a Neumann boundary condition. For each simulation, upwind interfacial averaging was used for fluid densities and relative permeabilities. A time-step increment factor of 1.25 was applied after convergence.
The simulated (modified STOMP) NAPL saturations at t=12 hours, 10 and 20 days are shown in Figures 1-3, respectively.

Example 3

For reasons of comparison an experiment has been carried out in accordance with a known STOMP simulation method. This simulation experiment was carried out in the similar manner as Example 2, except that an unmodified STOMP was used in which:

   1) Non-spreading behavior is not included. STOMP assumes that free NAPL is always continuous and located between the aqueous and gaseous phase.
   2) The permeability is described according to equation 1.
   3) The interfacial tension had to chosen and party adjusted in such a way that:

$$\sigma_{gl} = \sigma_{nl} + \sigma_{gn}$$

   where σ is the interfacial tension and the subscripts gl,nl and gn refer to gaseous-aqueous, NAPL-aqueous and gaseous-NAPL, respectively.
   The simulated NAPl saturations (unmodified STOMP) for t=12 hours, 10 and 20 days are shown in Figures 1,2, and 3, respectively.

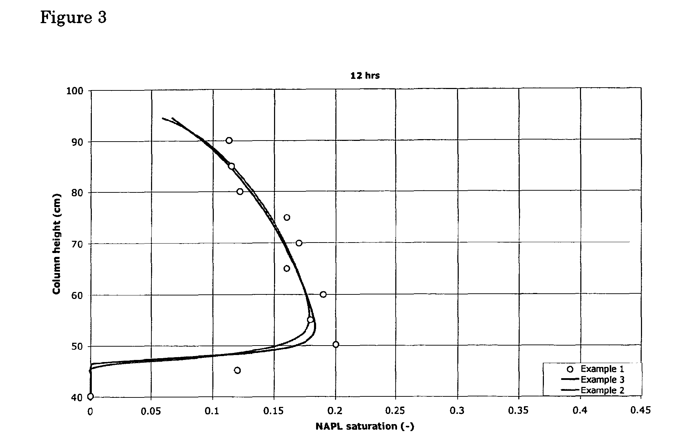

In Figures 1-3, the results of the numerical experiments of Examples 2 and 3 are compared against the measured results obtained in Example 1. Figure 1 shows that the results of both simulation runs compare well with the measured data of Example 3 during this part of the infiltration, when free NAPL saturations are well above the CDOS. At t=10 and T=20 days, however, the simulated profile of the amount of CCL$_4$ with the known method according to Example 3 is significantly lower than both the results obtained in Examples 1 and 2. An even more important effect is observed when the results in Figures 2 and 3 are compared with each other. The simulated NAPL saturation using the known method of Example 3 has continued to decrease, whereas both the actual measurements and the results obtained with the method of the present invention (Example 2) are constant between t=10 and 20 days. The decrease of the NAPL saturation in the simulations according to the known method will continue and deviations from the measured values will grow with time. In view of the above, it will be clear that the method in accordance with the present invention constitutes a considerable improvement when compared with the simulation method known in the art.

References

1. Brooks, R.H. and Corey, A.T. (1964). Hydraulic properties of porous media. In Hydrology Papers, Fort Collins: Colorado State University.

2. Corey, A.T. (1994) Mechanics of immiscible fluids in porous media. Water resources, Highlands Ranch, Colo.

3. Hofstee, C., Dane, J.H. and Hill, W.E. (1997a). Three-fluid retention in porous media involving water, PCE and air. J. Contam Hydrol. 25: 235-247.

4. Hofstee, C., M. Oostrom, J.H. Dane, and R.C. Walker. (1997b). Infiltration and redistribution of perchloroethylene in partially saturated, stratified porous media. J. Contam. Hydrol. 34: 293-313.

5. Kaluarachchi, JJ and Parker, JC. (1992) Multiphase flow with a simplified model for oil entrapment, Transport in Porous Media 7: 1-14.

6. Leverett, M.C. and Lewis, W.B. (1941). Steady flow of gas-oil-water mixtures through unconfined sands. Pet. Trans. AIME 142: 107-116.

7. Mualem, Y. (1976). A new model for predicting the hydraulic conductivity of unsaturated porous media. Water Resources Research 12: 513-522.

8. Oostrom, M., Hofstee, C., Lenhard, R.J. and Wietsma, T.W. (2003) Flow behaviour and residual saturation formation of liquid carbon tetrachloride in unsaturated heterogeneous porous media. Journal of Contaminant Hydrology 64: 93-112.

9. Oostrom, M., White, M.D. and Brusseau, M.L. (2001). Theorectical estimation of free en entrapped wetting and nonwetting fluid interfacial areas in porous media. Adv. Water Resources 24: 887-898.

10. Purcell, W.R. (1949). Capillary pressure-their measurements using mercury and the calculation of the permeability therefrom. J. Pet. Technol. 1:39-46.

11. Van Genuchten, M.T. (1980) A closed-form equation for predicting the hydraulic conductivity of unsaturated soils. Soil Sci. Soc. Am. J. 44: 892-898.

12. White, M.D. and Oostrom, M. (2000a) Subsurface Transport Over Multiple Phases. Version 2.0. Theory guide, Pacific Northwest National Laboratory.

13. White, M.D. and Oostrom, M. (2000b) Subsurface Transport Over Multiple Phases. Version 2.0. User's guide, Pacific Northwest National Laboratory.

**Claims**

1. A method for predicting final organic saturation in a porous medium comprising an aqueous phase, a gaseous phase and an organic liquid phase, which method comprises the steps of:

a) providing a sample of the porous medium;

b) determining the relationship between the permeability, fluid saturation and capillary pressure of the sample;

c) determining the critical detached organic saturation (CDOS) of the sample at a particular aqueous or gaseous saturation, whereby the CDOS is defined as being the critical continuous organic saturation at the continuous aqueous gas interface at which the relative permeability of the gaseous phase of the sample falls abruptly to zero;

d) estimating the interfacial area between the continuous aqueous and gaseous phases as a function of the aqueous or gaseous saturation;

e) determining the ratio of the CDOS over the area of the interface between the aqueous and gaseous phases;

f) performing a simulation of the distribution of the aqueous and gaseous phases using a continuum based multi-fluid flow model;

g) determining for each time step and note of the simulation whether the organic liquid phase saturation exceeds the CDOS at the particular aqueous or gaseous saturation;

h) set the relative permeability of the organic liquid phase to zero, when the CDOS has been reached; and

i) continue the simulation of the distribution of the aqueous and gaseous phases and maintain the relative permeability of the organic liquid phase at zero as long as the CDOS is not exceeded.

**2.** A method according to claim 1, wherein the porous medium comprises an undisturbed soil sample, a rock core or an artificially packed column or flow cell.

**3.** A method according to claim 1 or 2, wherein in step (b) the relationship between the permeability, the fluid saturation and capillary pressure of the sample is determined by means of a combination of a column experiment and scaling techniques, or a combination of techniques such as pressure cells, suction apparatuses, mercury intrusion and centrifuges..

**4.** A method according to any one of claims 1 to 3, wherein in step (c) the critical detached organic saturation of the sample at a particular aqueous or gaseous saturation is determined by means of an experiment or estimation based on the aforementioned relationship between the capillary pressure and the fluid saturation.

**5.** A method according to any one of claims 1-4, wherein in step (d) the interfacial area between the continuous aqueous and gaseous phases as a function of the aqueous or gaseous saturation is estimated by means of the following an expression:

$$A_{nl}^f = \frac{(1-S_m)n_D}{\sigma_{nl}} \int_{\overline{S_l}}^{1} P_{cnl} d\overline{\overline{S_l}} \quad (2)$$

**6.** A method according to any one of claims 1-4, wherein in step (e) the ratio of the CDOS over the area of the interface between the aqueous and gaseous phases is determined by means of a division between the measured CDOS and the estimated interfacial area between the continuous aqueous and gaseous phase at the corresponding aqueous saturation.

**7.** A method according to any one of claims 1-6, wherein in step (f) the continuum based multi-fluid flow model comprises the concept of CDOS which is written as

$$\overline{S_{nd}^c} = \overline{S_{nd}^{\max}}(1 - \overline{\overline{S_l}}^{\left(1-\frac{1}{\lambda}\right)}) \quad (5)$$

**8.** A method according to any one of claims 1-7, wherein in step (g) use is made of the simulated NAPL -and aqueous saturations to determine for each time step and note of the simulation whether the organic liquid phase saturation exceeds the CDOS at this particular aqueous or gaseous saturation.

**9.** A process for recovering an organic liquid phase from a subsurface formation or for predicting the amount of organic contaminants in the vadose zone, in which process use is made of a method according to any one of claims 1-8 for predicting the quantity of final organic liquid phase present in the subsurface formation.

**10.** A process according to claim 9, wherein the subsurface formation comprises an oil reservoir or an unsaturated zone.

## Figure 1

20 days — NAPL saturation (-) vs Column height (cm)

Legend: O Example 1; Example 3; Example 2

## Figure 2

10 days — NAPL saturation (-) vs Column height (cm)

Legend: O Example 1; Example 3; Example 2

Figure 3

**European Patent Office**

## EUROPEAN SEARCH REPORT

Application Number

EP 04 07 6931

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | LENHARD ET AL: "A constitutive model for air-NAPL-water flow in the vadose zone accounting for immobile, non-occluded (residual) NAPL in strongly water-wet porous media" JOURNAL OF CONTAMINANT HYDROLOGY, vol. 71, July 2004 (2004-07), pages 261-282, XP002310869 Available online 16 March 2004 * abstract * * page 268, paragraph 3.3 - page 270, paragraph 3.4 * * page 279, paragraph 5. - page 280 * | 1-10 | G01N33/24 G01N15/08 |
| A | OOSTROM M; LENHARD R J: "Carbon Tetrachloride Flow Behavior in Unsaturated Hanford Caliche Material: An investigation of Residual Nonaqeous Phase Liquid" VADOSE JONE JOURNAL, vol. 2, February 2003 (2003-02), pages 25-33, XP002310870 * abstract * * page 27, column 2 - page 29, column 1 * | 1-10 | |
| A,D | OOSTROM ET AL: "Flow behaviour and residual saturation formation of liquid carbon tetrachloride in unsaturated heterogeneous porous media" JOURNAL OF CONTAMINANT HYDROLOGY, vol. 64, June 2003 (2003-06), pages 93-112, XP002310871 Available online 05 April 2003 * abstract * * page 102, paragraph 3.4 * * page 110, last paragraph * | 1-10 | TECHNICAL FIELDS SEARCHED (Int.Cl.7) G01N |

-/--

The present search report has been drawn up for all claims

| Place of search | Date of completion of the search | Examiner |
|---|---|---|
| Munich | 15 December 2004 | Bravin, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 04 07 6931

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | OOSTROM ET AL: "Theoretical estimation of free and entrapped nonwetting-wetting fluid interfacial areas in porous media" ADVANCES IN WATER RESOURCES, vol. 24, August 2001 (2001-08), pages 887-898, XP002310872 Available online 22 June 2001 * abstract * * page 892, column 1, paragraph 2.2 - column 2, paragraph 3 * * page 896, column 2, paragraph 6 - page 897, column 2 * ----- | 1-10 | |
| | | | TECHNICAL FIELDS SEARCHED (Int.Cl.7) |

The present search report has been drawn up for all claims

| Place of search | Date of completion of the search | Examiner |
|---|---|---|
| Munich | 15 December 2004 | Bravin, M |

EPO FORM 1503 03.82 (P04C01)